# EUROPEAN PATENT SPECIFICATION

(11) **EP 4 120 051 B1**
(45) Date of publication and mention of the grant of the patent: **08.04.2026**
(21) Application number: 21185542.4
(22) Date of filing: 14.07.2021
(51) Int. Cl.: G06F 3/01, A61B 5/11, A61B 5/00, G02B 27/01, G02C 5/00, G02C 11/00

(54) **OPTO-ELECTRONIC DEVICE AND METHOD FOR PROVIDING VISUAL ORIENTATION**
OPTOELEKTRONISCHE VORRICHTUNG UND VERFAHREN ZUR BEREITSTELLUNG OPTISCHER ORIENTIERUNG
DISPOSITIF OPTO-ÉLECTRONIQUE ET PROCÉDÉ PERMETTANT DE FOURNIR UNE ORIENTATION VISUELLE

(43) Date of publication of application: 18.01.2023
(73) Proprietor: Universität Konstanz, 78464 Konstanz (DE)
(72) Inventor: Assländer, Lorenz, 78462 Konstanz (DE); Streuber, Stephan, 78462 Konstanz (DE)
(74) Representative: Altmann Stößel Dick Patentanwälte PartG mbB

(56) References cited:
- JP-A- 2008 256 946
- US-A- 5 966 680
- US-A1- 2002 099 257
- US-A1- 2015 273 179
- WESLEY W.O. KRUEGER: "Controlling motion sickness and spatial disorientation and enhancing vestibular rehabilitation with a user-worn see-through display", THE LARYNGOSCOPE, vol. 121, no. S2, 22 December 2010 (2010-12-22), United States, pages S17 - S35, XP055453221, ISSN: 0023-852X, DOI: 10.1002/lary.21373

## Description

### Technical Field

The present invention relates to an opto-electronic device and to a method for providing visual orientation.

### Background art

Older adults and some patient populations are at increased risk of falling, with a high probability of those falls resulting in injuries. Such falls are often caused by a bad visual reference such as darkness, low contrast or a moving scene. Losing a focus on a visual reference for only a fraction of a second is enough to cause a fall. Falls and fall related injuries negatively impact the ability of older individuals to perform daily tasks, and substantially impact health care costs. Less easily quantified, but arguably more important, is the reduced quality of life from fall related injuries: disability, dependence on others, lost time from work or household duties, and self-restricted social interactions due to fear of falling. It is essential to identify affordable solutions to this growing medical, social, and economic problem that are easily accessible to a large segment of the aging population. Most falls occur during dynamic activities like walking or transitions from sitting/standing to walking, yet most visual biofeedback for postural control is provided during standing. Thus, falls are a major health problem for individuals and the society. Particularly, the risk of fall significantly increases for human beings as from an age of 65 years. Technical systems to improve balance can contribute to reduce the risk of falling.

Some approaches attempt to use vibrating devices or other stimulations of the skin to improve balance and can prevent falls or postural instability such as disclosed in U.S. Patent Application No. US 2010/286571 A1 or in U.S. Patent No. 8,974,402 B2.

Another attempt is to improve balance in subjects that have no functioning vestibular organs.

Here, electrical stimulations of the vestibular system are used to provide additional sensory information. The technique, called electric or galvanic vestibular stimulation, generates a sensation of head roll (left and right rotation of the head), that evokes corresponding balance responses. Such a stimulation can in principle be applied through implants similar to a cochlea implant, or externally by electrodes attached to the mastoid process behind the ear. In implants, also 3D feedback is possible. Examples for this approach are disclosed in U.S.

Patent No. 8,868,202 B2, U.S. Patent No. 6,546,291 B2 and U.S. Patent No. 8,855,774 B2.

Some research groups try to develop early warning systems. A device, e.g. smart phone, is programmed to detect when a fall is occurring and provide some kind of warning, e.g. by vibrating or playing a sound.

US 5,966,680 A discloses a device and method which operates as an artificial labyrinth to eliminate sensory mismatch between the natural labyrinth/vestibular system and the vision system of an individual. The method can be effected by means of a device which senses true body orientation and displays corresponding visual orientation cues that the brain can use to confirm other visual position information. The display can be projected into space in front of the user, directly onto the user's retina, or effected by pictorial scene averaging.

US 2002/0099257 A1 discloses a head-mounted display wherein the display of an independent visual background provides a visual reference corresponding to the perceptions of a person's vestibular system, thereby substantially reducing or eliminating motion sickness that otherwise occur due to a mismatch between the visual perception of motion or non-motion and the sensations of the vestibular system.

JP 2008-256946 A discloses a motion sickness prevention apparatus for a head-mounted display device comprising a visual cue production unit to produce visual cues having a fixed position with respect to world coordinates using at the periphery of the user's visual field.

One available product is the so called boarding glasses which use transparent rings filled with coloured liquid. When tilting the head, the liquid provides a kind of visual horizon.

Despite the advantages provided by these devices and methods described above, there are still some drawbacks.

Early warning systems have two major drawbacks. Firstly, robust online fall detection is extremely difficult to achieve and secondly, by concept already comes too late, i.e. when the fall is already in progress. It is unlikely that a fall can be stopped, while it is already occurring. Augmentation or substitution of vestibular sensory input through galvanic vestibular stimulation has several drawbacks. It is restricted to medio-lateral information (leftright). It is not very user friendly, since either electrodes need to be attached on a daily basis and implants are highly invasive. Other techniques such as sensory augmentation through vibrations at the trunk have been proposed since many years, but have not delivered convincing results, i.e. they do not work properly. Boarding glasses are designed as an aid against motion sickness. They have not been used as a balance aid for dynamic situations and would have several drawbacks, such as reduced field of view, no feedback in dark situations, jerky movement of the coloured water due to water inertia in dynamic situations, such as walking. Goggles and contact lenses improve visual acuity and therefore also improve balance. However, they only improve the reference of an existing visual scene and do not substitute orientation cues when no visual reference is available (darkness or viewing a scene with little contrast, such as a white wall) or in conflict with other sensory cues (moving scene, e.g. a bus). Augmented reality glasses have the capacity to provide additional visual orientation cues. However, the devices are not designed to be worn in everyday life or as add-ons to goggles. Furthermore, they are designed to provide additional information for other purposes than balance in the central field of view. The central field of view should remain undisturbed for interacting with the surrounding. The peripheral field of view strongly contributes to ego-motion sensation, allows the user to have an undisturbed central field of view and is not targeted by state of the art augmented reality devices.

### Problem to be solved

It is therefore desirable to provide a technical device and a method that can improve visual orientation and reduce the risk of falling.

### Summary

This problem is addressed by an opto-electronic device and a method for providing visual orientation with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may all refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention, there is provided an opto-electronic device. The opto-electronic device comprises at least one sensor configured to obtain head orientation data including information on an orientation of a user's head. The opto-electronic device further comprises a processing unit configured to process the head orientation data so as to obtain self motion data including information on the user's self motion and to provide at least one visual cue based on the obtained self motion data. The opto-electronic device further comprises a display device configured to display the at least one visual cue at a display at least in a periphery of a visual field of the user.

Thus, the sensor allows to extract or acquire information on a head orientation with respect to a surrounding space or room. The processing unit allows to extract or acquire self motion information from the sensor data and transform it to visual cues. The display device allows to project or otherwise visually illustrate the visual cues to the user at the display. The display may be a device separate from the opto-electronic device or may alternatively be part of the opto-electronic device. Thus, the display provides a sense of self motion and orientation with respect to the room or space vertical. Particularly, the displayed visual cues in the periphery of the visual field of the user are used to provide additional space veridical visual orientation cues for the user. This visual reference is integrated by the human balance control mechanism. Thereby, falls can be prevented by improving the user's balance. The opto-electronic device will particularly benefit balance in situations with a bad visual reference.

The sensor may include at least an inertial measurement unit and/or a camera and/or a LIDAR device configured to obtain the head orientation data and head movement data including information on a movement of the user's head. Thus, not only information on a head orientation but also on its movement within a given space or room may be acquired allowing to precisely adapt the visual cue to a given orientation and/or movement situation of the user.

The display device may be configured to display the visual cue at the display as a virtually stationary foreground with a predetermined distance to the user. Thus, when moving the head in space, e.g. rotation or translation, the projected visual cue corrects for this motion, such that the visual reference is virtually space stationary from the user's perspective. This space stationary visual reference is integrated by the human balance control mechanism so as to increase the user's balance. The virtually space stationary foreground represents a kind of fixed scene for the user arranged at the predetermined distance.

The display device may be further configured to provide the visual cue with an optical flow. Thereby, a potential distraction or irritation of the user is avoided.

The display device may be further configured to vary a size at least of first parts of the visual cue and to fade in or fade out at least of second parts of the visual cue so as to provide the optical flow. The optical flow may be created by expansion or contraction of the visual cue. For example, the displayed visual cue includes a certain pattern which expands and fades in while new parts of the pattern are created, which in turn expand and fade. Thus, a continuous pattern is realized.

The display device may be further configured to gradually vary the size of the first parts of the visual cue and to fade in or fade out the second parts of the visual cue so as to provide a gradual optical flow. Thus, the optical flow is realized as a continuous optical flow.

The display device may be further configured to expand the first parts of the visual cue and to fade in the second parts of the visual cue if a distance from the visual cue to the user decreases, and wherein the display device is configured to contract the first parts of the visual cue and to fade out the second parts of the visual cue if a distance from the visual cue to the user increases. Thus, a kind of approaching and departing from the visual cue is imitated for the user.

The first parts and the second parts of the visual cue may be different from one another or are identical to one another. Thus, identical or different portions may be displayed.

The display device may be configured to display the visual cue so as to form a contrast relative to a background in the visual field of the user. Thus, the orientation for the user is further improved as the visual cue is clearly visible. For example, the visual cue may be rather dark if the background is rather bright and *vice versa.*

The visual cue may be configured to provide parallax information to the user. Thus, some kind of depth or spatial information is provided.

The visual cue may be a space veridical orientation cue. Thus, the visual cue may be simulated as a real world part or portion.

The visual cue may be at least one cue selected from the group consisting of: horizontal lines, vertical lines, a shape of a room, a shape of a tunnel, a dot pattern, a checked pattern and a pattern with depth information. Thus, different types of cues are suitable to correct a motion of a user's head.

The display device may be configured to vary the visual cue based on the obtained self motion data. Thus, the visual cue may be adapted to the respective motion.

The display device may be configured to limit a shifting of the visual cue to maximum 4°/s. Thus, a displacement of the visual cue with a speed exceeding the maximum resolution of the human visual system is prevented. Thus, for high velocities, a kind of saturation is integrated.

The display device may be configured to adjust a resolution of the visual cue based on a distance of the visual cue from a user's fovea. Thus, a sharp picture is displayed for the most important portions of the visual field of the user.

The display device may be configured to increase the resolution of the visual cue based on a decreasing distance of the visual cue from the user's fovea. Thus, the resolution is the higher the closer to the fovea it is presented.

The processing unit may be configured to provide a plurality of visual cues based on the obtained self motion data, and the display device may be configured to display the plurality of visual cues at the display at least in the periphery of the visual field of the user, wherein the visual cues may be identical to one another or may be different from one another. Thus, more than one visual cue may be displayed increasing the orientation information for the user.

The display device may be configured to display the plurality of visual cues at the display in a subsequent order or at the same time. Thus, the visual cues may be adapted to different point in time of a motion of the user's head.

The display may be a transparent screen or a user's retina. Thus, the visual field is not obstructed.

The opto-electronic device may be configured to be integrated into or attached to a visual aid or is formed as smart glasses. Thus, the opto-electronic device may be integrated into an already existing visual aid such as glasses or may be designed as a stand-alone solution.

In a further aspect of the invention, there is provided a method for providing visual orientation. The method comprises the following steps, preferably in the given order:
- obtaining head orientation data including information on an orientation of a user's head,
- processing the head orientation data so as to obtain self motion data including information on the user's self motion and providing at least one visual cue based on the obtained self motion data, and
- displaying the at least one visual cue at a display at least in a periphery of a visual field of the user.

Thus, information on a head orientation with respect to a surrounding space or room are extracted or acquired. Further, self motion information are extracted or acquired and transformed to visual cues. The thus provided visual cue is projected or otherwise visually illustrated to the user. Thus, a sense of self motion and orientation with respect to the room or space vertical is provided. Particularly, the displayed visual cues in the periphery of the visual field of the user are used to provide additional space veridical visual orientation cues for the user. This visual reference is integrated by the human balance control mechanism. Thereby, falls can be prevented by improving the user's balance.

The method may further comprise displaying the visual cue as a virtually stationary foreground with a predetermined distance to the user. Thus, when moving the head in space, e.g. rotation or translation, the projected visual cue correct for this motion, such that the visual reference is virtually space stationary. This space stationary visual reference is integrated by the human balance control mechanism and increases the user's balance. The virtually space stationary foreground represents a kind of fixed scene for the user arranged at the predetermined distance.

The method may further comprise providing the visual cue with an optical flow. Thereby, a potential distraction or irritation of the user is avoided.

The method may further comprise varying a size at least of first parts of the visual cue and fading in or fading out at least of second parts of the visual cue so as to provide the optical flow. The optical flow may be created by expansion or contraction of the visual cue. For example, the displayed visual cue includes a certain pattern which expands and fades in while new parts of the pattern are created, which in turn expand and fade. Thus, a continuous pattern is realized.

The method may further comprise gradually varying a size at least of first parts of the visual cue and gradually fading in or fading out at least of second parts of the visual cue so as to provide a gradual optical flow. Thus, the optical flow is realized as a continuous optical flow.

The first parts and the second parts of the visual cue may be different from one another or are identical to one another. Thus, identical or different portions may be displayed.

The method may further comprise displaying the visual cue so as to form a contrast relative to a background in the visual field of the user. Thus, the orientation for the user is further improved as the visual cue is clearly visible. For example, the visual cue may be rather dark if the background is rather bright and *vice versa.*

The method may further comprise providing parallax information to the user by means of the visual cue. Thus, some kind of depth or spatial information is provided.

The visual cue may be a space veridical orientation cue. Thus, the visual cue may simulated as a real world part or portion.

The visual cue may be at least one cue selected from the group consisting of: horizontal lines, vertical lines, a shape of a room, a shape of a tunnel, a dot pattern, a checked pattern and a pattern with depth information. Thus, different types of cues are suitable to correct a motion of a user's head.

The method may further comprise varying the visual cue based on the obtained self motion data. Thus, the visual cue may be adapted to the respective motion.

The method may further comprise limiting a shifting of the visual cue to maximum 4°/s. Thus, a displacement of the visual cue with a speed exceeding the maximum resolution is prevented. Thus, for high velocities, a kind of saturation is integrated.

The method may further comprise adjusting a resolution of the visual cue based on a distance of the visual cue from a user's fovea. Thus, a sharp picture is displayed for the most important portions of the visual field of the user.

The method may further comprise increasing the resolution of the visual cue based on a decreasing distance of the visual cue from the user's fovea. Thus, the resolution is the higher the closer the distance is to the fovea.

The method may further comprise providing a plurality of visual cues based on the obtained self motion data, and displaying the plurality of visual cues at the display at least in the periphery of the visual field of the user, wherein the visual cues are identical to one another or are different from one another. Thus, more than one visual cue may be displayed increasing the orientation information for the user.

The method may further comprise displaying the plurality of visual cues at the display in a subsequent order or at the same time. Thus, the visual cues may be adapted to different point in time of a motion of the user's head.

The method may be computer-implemented.

In a further aspect of the invention, there is provided a computer program comprising instructions which, when the program is executed by the opto-electronic device according to any one of the preceding claims referring to an opto-electronic device, cause the opto-electronic device to perform the method according to any one of the preceding claims referring to a method.

In a further aspect of the invention, there is provided a computer-readable storage medium comprising instructions which, when the program is executed by the opto-electronic device according to any one of the preceding claims referring to an opto-electronic device, cause the opto-electronic device to perform the method according to any one of the preceding claims referring to a method.

The term "opto-electronic device" as used herein is a broad term and is to be given its ordinary and customary meaning to a person of ordinary skill in the art and is not to be limited to a special or customized meaning. The term specifically may refer, without limitation, to a device that is configured to source, detect and control light, particularly light visible to human beings. Opto-electronic devices are electrical-to-optical or optical-to-electrical transducers, or instruments that use such devices in their operation. Particularly, the term may refer to a device that is configured to add visible information alongside or to what the wearer sees. Superimposing information onto a field of view may be achieved through an optical head-mounted display or embedded wireless glasses with transparent heads-up display or augmented reality overlay. These systems have the capability to reflect projected digital images as well as allow the user to see through it or see better with it.

The term "head orientation" and its equivalents as used herein is a broad term and is to be given its ordinary and customary meaning to a person of ordinary skill in the art and is not to be limited to a special or customized meaning. The term specifically may refer, without limitation, to the orientation of a human head within a surrounding three-dimensional space or room. The orientation may be expressed as an orientation relative to given reference markers.

The term "processing unit" as used herein is a broad term and is to be given its ordinary and customary meaning to a person of ordinary skill in the art and is not to be limited to a special or customized meaning. The term specifically may refer, without limitation, to an arbitrary device adapted to process the head orientation data and to transform it into visual cues, preferably by using at least one processing device and/or at least one application-specific integrated circuit. Thus, as an example, the processing unit may comprise one or more programmable devices such as one or more computers, application-specific integrated circuits (ASICs), Field Programmable Gate Arrays (FPGAs), or other devices which are configured for performing the above-mentioned process. Thus, as an example, the at least one processing unit may have a software code stored thereon comprising a number of computer commands. The processing unit may provide one or more hardware elements for performing one or more of the named operations and/or may provide one or more processing units with software running thereon for performing one or more of the named operations.

The term "display device" as used herein is a broad term and is to be given its ordinary and customary meaning to a person of ordinary skill in the art and is not to be limited to a special or customized meaning. The term specifically may refer, without limitation, to an output device for presentation of information in visual at a display. When the input information that is supplied has an electrical signal the display device is called an electronic display device.

The term "self motion" as used herein is a broad term and is to be given its ordinary and customary meaning to a person of ordinary skill in the art and is not to be limited to a special or customized meaning. The term specifically may refer, without limitation, to the own motion of a user's head. The motion may relate to a translatory and/or rotational motion.

The term "visual cue" as used herein is a broad term and is to be given its ordinary and customary meaning to a person of ordinary skill in the art and is not to be limited to a special or customized meaning. The term specifically may refer, without limitation, to a statistic or signal that can be extracted from the sensory input by a perceiver, that indicates the state of some property of the world that the perceiver is interested in perceiving. The cue is visible to the perceiver.

The term "visual field" as used herein is a broad term and is to be given its ordinary and customary meaning to a person of ordinary skill in the art and is not to be limited to a special or customized meaning. The term specifically may refer, without limitation, to a field of view. Particularly, the term may refer to the extent of the observable world that is seen at any given moment by a human being. Thus, a visual field may refer to the spatial array of visual sensations available to observation in introspectionist psychological experiments. Or simply, visual field can be defined as the entire area that can be seen when an eye is fixed straight at a point. The normal (monocular) human visual field extends to approximately 60 degrees nasally (toward the nose, or inward) from the vertical meridian in each eye, to 107 degrees temporally (away from the nose, or outwards) from the vertical meridian, and approximately 70 degrees above and 80 below the horizontal meridian. The binocular visual field is the superimposition of the two monocular fields. **In** the binocular field, the area left of the vertical meridian is referred to as the left visual field (which is temporally for the left, and nasally for the right eye); a corresponding definition holds for the right visual field. The four areas delimited by the vertical and horizontal meridian are referred to as upper/lower left/right quadrants. In the European Union, the minimum field requirement for driving is 50 degrees to either side of the vertical meridian and 120 degrees horizontally in total, and 20 degrees above and below the horizontal meridian. The macula corresponds to the central 17 degrees diameter of the visual field; the fovea to the central 5.2 degrees, and the foveola to 1-1.2 degrees diameter. The periphery of the visual field is the area adjacent the outer border of the visual field.

The term "periphery of a visual field" as used herein is a broad term and is to be given its ordinary and customary meaning to a person of ordinary skill in the art and is not to be limited to a special or customized meaning. The term specifically may refer, without limitation, to the portion, area or part of the visual field outside the point of fixation, i.e. away from the center of gaze or, when viewed at large angles, in (or out of) the "corner of one's eye". The vast majority of the area in the visual field is included in the notion of peripheral vision. "Far peripheral" vision refers to the area at the edges of the visual field, "mid-peripheral" vision refers to medium eccentricities, and "near-peripheral", sometimes referred to as "para-central" vision, exists adj acent to the center of gaze. The inner boundaries of periphery of the visual field can be defined in any of several ways depending on the context. In everyday language the term "peripheral vision" is often used to refer to what in technical usage would be called "far peripheral vision." This is vision outside of the range of stereoscopic vision. It can be conceived as bounded at the center by a circle 60° in radius or 120° in diameter, centered around the fixation point, i.e., the point at which one's gaze is directed. However, in common usage, periphery of the visual field may also refer to the area outside a circle 30° in radius or 60° in diameter. In vision-related fields such as physiology, ophthalmology, optometry, or vision science in general, the inner boundaries of peripheral vision are defined more narrowly in terms of one of several anatomical regions of the central retina, in particular the fovea and the macula. The periphery of a visual field is not imaged onto the central part of the retina, i.e. the fovea.

The term "display" as used herein is a broad term and is to be given its ordinary and customary meaning to a person of ordinary skill in the art and is not to be limited to a special or customized meaning. The term specifically may refer, without limitation, to any device suitable to provide an illustrative or visual output. The term specifically may refer to an electronic visual display, informally a screen, for presentation of images, text, or video transmitted electronically, without producing a permanent record. The term specifically may refer, without limitation, to a natural display such as a retina of an eye of a human being.

The term "inertial measurement unit" as used herein is a broad term and is to be given its ordinary and customary meaning to a person of ordinary skill in the art and is not to be limited to a special or customized meaning. The term specifically may refer, without limitation, to an electronic device that measures and reports a body's specific force, angular rate, and sometimes the orientation of the body, using a combination of accelerometers, gyroscopes, and sometimes magnetometers. An inertial measurement unit works by detecting linear acceleration using one or more accelerometers and rotational rate using one or more gyroscopes. Some also include a magnetometer which is commonly used as a heading reference. Typical configurations contain one accelerometer, gyro, and magnetometer per axis for each of the three principal axes: pitch, roll and yaw.

The term "LIDAR device" as used herein is a broad term and is to be given its ordinary and customary meaning to a person of ordinary skill in the art and is not to be limited to a special or customized meaning. The term specifically may refer, without limitation, to a device configured to determine ranges (variable distance) by targeting an object with a laser and measuring the time for the reflected light to return to the receiver. LIDAR can also be used to make digital 3-D representations of areas, due to differences in laser return times, and by varying laser wavelengths. LIDAR is an acronym of "light detection and ranging". LIDAR uses ultraviolet, visible, or near infrared light to image objects. It can target a wide range of materials, including non-metallic objects, rocks, rain, chemical compounds, aerosols, clouds and even single molecules. A narrow laser beam can map physical features with very high resolutions; for example, an aircraft can map terrain at 30-centimetre (12 in) resolution or better. Wavelengths vary to suit the target: from about 10 micrometers (infrared) to approximately 250 nm (UV). Typically, light is reflected via backscattering, as opposed to pure reflection one might find with a mirror. Different types of scattering are used for different LIDAR applications: most commonly Rayleigh scattering, Mie scattering, Raman scattering, and fluorescence. Suitable combinations of wavelengths can allow for remote mapping of atmospheric contents by identifying wavelength-dependent changes in the intensity of the returned signal.

The term "virtually stationary" as used herein is a broad term and is to be given its ordinary and customary meaning to a person of ordinary skill in the art and is not to be limited to a special or customized meaning. The term specifically may refer, without limitation, to a situation where the displayed visual cue is superimposed to a background scene and remains at its position in front of the background scene independent on whether the visual field and, thereby the background, is shifted by a movement of the user's head.

The term "space veridical visual orientation cue" as used herein is a broad term and is to be given its ordinary and customary meaning to a person of ordinary skill in the art and is not to be limited to a special or customized meaning. The term specifically may refer, without limitation, to a type of cue that allows orientation for the user within a real three dimensional space. The cues are designed to improve balance in static and dynamic conditions, e.g. walking. The term "space veridical" as used herein may refer to a horizontal orientation or a vertical orientation with respect to a direction of gravity.

The term "parallax information" as used herein is a broad term and is to be given its ordinary and customary meaning to a person of ordinary skill in the art and is not to be limited to a special or customized meaning. The term specifically may refer, without limitation, to information representing a parallax effect. Parallax refers to a displacement or difference in the apparent position of an object viewed along two different lines of sight, and is measured by the angle or semi-angle of inclination between those two lines. Due to foreshortening, nearby objects show a larger parallax than farther objects when observed from different positions, so parallax can be used to determine distances.

The term "smart glasses" as used herein is a broad term and is to be given its ordinary and customary meaning to a person of ordinary skill in the art and is not to be limited to a special or customized meaning. The term specifically may refer, without limitation, to wearable computer glasses that add information alongside or to what the wearer sees. Alternatively, smart glasses are sometimes defined as wearable computer glasses that are able to change their optical properties at runtime. Smart sunglasses which are programmed to change tint by electronic means are an example of the latter type of smart glasses. Superimposing information onto a field of view is achieved through an optical head-mounted display (OHMD) or embedded wireless glasses with transparent heads-up display (HUD) or augmented reality (AR) overlay. These systems have the capability to reflect projected digital images as well as allow the user to see through it or see better with it. While early models can perform basic tasks, such as serving as a front end display for a remote system, as in the case of smart glasses utilizing cellular technology or Wi-Fi, modern smart glasses are effectively wearable computers which can run self-contained mobile apps. Some are handsfree and can communicate with the Internet via natural language voice commands, while others use touch buttons. Like other computers, smart glasses may collect information from internal or external sensors. It may control or retrieve data from other instruments or computers. It may support wireless technologies like Bluetooth, Wi-Fi, and GPS. A small number of models run a mobile operating system and function as portable media players to send audio and video files to the user via a Bluetooth or Wi-Fi headset. Some smart glasses models also feature full lifelogging and activity tracker capability.

The term "computer-implemented" as used herein is a broad term and is to be given its ordinary and customary meaning to a person of ordinary skill in the art and is not to be limited to a special or customized meaning. The term specifically may refer, without limitation, to a process which is fully or partially implemented by using a data processing means, such as data processing means comprising at least one processing device. The term "computer", thus, may generally refer to a device or to a combination or network of devices having at least one data processing means such as at least one processing unit. The computer, additionally, may comprise one or more further components, such as at least one of a data storage device, an electronic interface or a human-machine interface.

Further disclosed and proposed herein is a computer program including computer-executable instructions for performing the method according to the present invention in one or more of the embodiments enclosed herein when the program is executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a schematic illustration of setup of an opto-electronic device according to an embodiment of the present invention;
- Figure 2: shows a perspective view of the inertial measurement unit;
- Figure 3: shows a perspective view of the processing unit;
- Figure 4: shows an example for visual cues;
- Figure 5: shows an example for the provision of the optical flow;
- Figure 6: shows a perspective view of a visual aid;
- Figure 7: shows a flowchart of a method for providing visual orientation.

### Detailed description of the embodiments

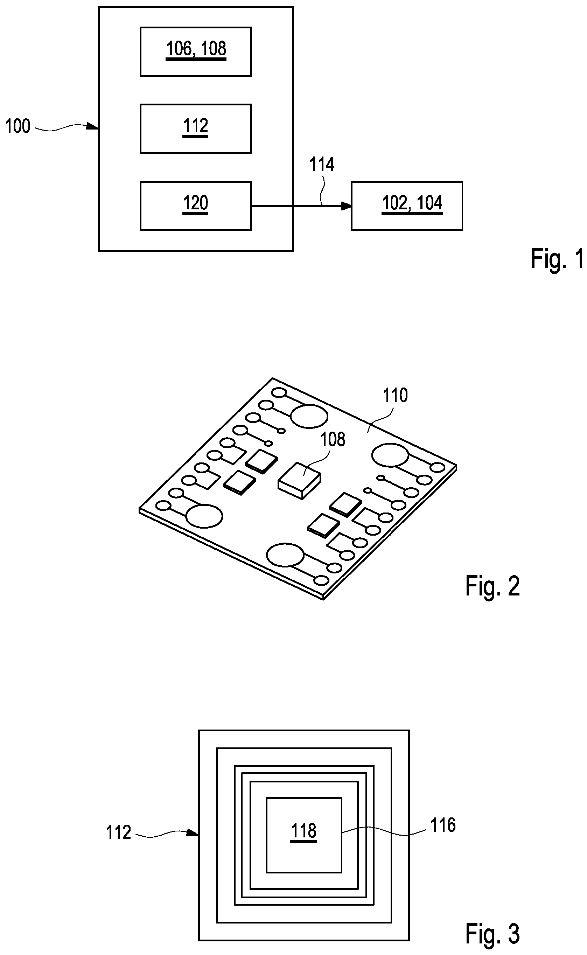

Figure 1 shows a schematic illustration of setup of an opto-electronic device 100 according to an embodiment of the present invention. Particularly, Figure 1 shows a block diagram of the components of the opto-electronic device 100. The opto-electronic device 100 is configured to be used together with a display 102. The display 102 is a transparent screen 104. The display 102 is a device separate from the opto-electronic device 100. It is explicitly stated that the display 102 may alternatively be part of the opto-electronic device 100. With other words, the opto-electronic device 100 and the display 102 may be integrated as one unit.

The opto-electronic device 100 further comprises at least one sensor 106 configured to obtain head orientation data including information on an orientation of a user's head. The sensor 106 includes at least an inertial measurement unit 108.

Figure 2 shows a perspective view of the inertial measurement unit 108. As shown in Figure 2, the inertial measurement unit 108 may be disposed on a printed circuit board 110. The inertial measurement unit 108 is configured to obtain the head orientation data and head movement data including information on a movement of the user's head. Alternatively or in addition, the sensor 106 includes a camera and/or a LIDAR device configured to obtain the head orientation data and head movement data including information on a movement of the user's head.

The opto-electronic device 100 further comprises a processing unit 112 configured to process the head orientation data so as to obtain self motion data including information on the user's self motion.

Figure 3 shows a perspective view of the processing unit 112. As shown in Figure 3, the processing unit 112 includes a processing device 116 such as a microprocessor and an application-specific integrated circuit (ASIC) 118.

The processing unit 112 is further configured to provide at least one visual cue 114 based on the obtained self motion data. The visual cue 114 is configured to provide parallax information to the user. The provision of the parallax information may be realized in that the projection of the visual cue 114 onto the display 102 is moved inversely proportional to a self-motion of the user. This may also be realized by two patterns which are moved at different velocities relative to the user. For example, two or more superimposed projections are illustrated at the same time, wherein a first one is rather slow in the background and a rather fast one is in the foreground. Particularly, the visual cue 114 is a space veridical orientation cue. The visual cue 114 is at least one cue selected from the group consisting of: horizontal lines, vertical lines, a shape of a room, a shape of a tunnel, a dot pattern, a checked pattern and a pattern with depth information.

The opto-electronic device 100 further comprises a display device 120 configured to display the at least one visual cue 114 at the display 102 at least in a periphery of a visual field of the user. The display device 120 may be an output device configured to project the visual cue 114 onto the display 102 such as a projector. Particularly, the display device 120 is configured to display the visual cue 114 at the display 102 as a virtually stationary foreground with a predetermined distance to the user.

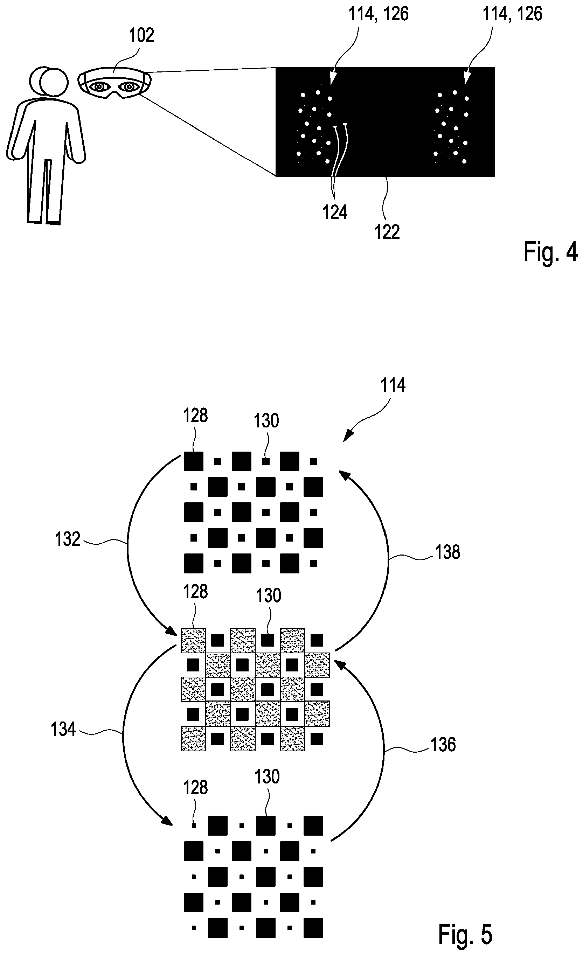

Figure 4 shows an example for visual cues 114 displayed by the display device 120. The display device 120 is configured to display the visual cue 114 so as to form a contrast relative to a background 122 in the visual field of the user. Figure 4 shows an example where the background 122 is rather dark and includes only two minor bright spots 124. Such a dark background scene is not suitable to provide visual orientation to the user. As is further shown in Figure 4, the present invention addresses this issue by displaying the visual cue 114 onto the display 102. Merely as an example, the displayed visual cue 114 includes a plurality of bright dots or spots 126. With other words, the visual cue 114 is a dot pattern, wherein the dots are arranged as an irregular pattern. Needless to say, the dots may be arranged as a regular pattern depending on the respective situation.

The display device 120 is further configured to provide the visual cue 114 with an optical flow. The display device 120 is configured to vary a size at least of first parts 128 of the visual cue 114 and to fade in or fade out at least of second parts 130 of the visual cue 114 so as to provide the optical flow. Particularly, the display device 120 is configured to gradually vary the size of the first parts 128 of the visual cue 114 and to fade in or fade out the second parts 130 of the visual cue 114 so as to provide a gradual optical flow. For this purpose, the display device 120 is configured to expand the first parts 128 of the visual cue 114 and to fade in the second parts 130 of the visual cue if a distance from the visual cue 114 to the user decreases. Further, the display device 120 is configured to contract the first parts 128 of the visual cue 114 and to fade out the second parts 130 of the visual cue 114 if a distance from the visual cue 114 to the user increases. The first parts 128 and the second parts 130 of the visual cue 114 may be different from one another or may be identical to one another.

Figure 5 shows an example for the provision of the optical flow by the display device 120. As shown in Figure 5, the example for the visual cue 114 comprises a plurality of squares arranged in a regular pattern. Needless to say, the following explanations apply to any other pattern as well unless otherwise stated. Figure 5 is divided in three portions representing exemplary steps for the provision of the optical flow. The provision of the optical flow will be described with the two steps arranged from the top to the bottom and two steps arranged from the bottom to the top. As shown in the upper portion of Figure 5, the visual cue 114 comprises first parts 128 and second parts 130, wherein in the upper portion, the first parts 128 are greater than the second parts 130. In order to provide the optical flow, the second parts 130 are expanded and the first parts 128 are faded out in a first step when proceeding towards the middle portion of Figure 5 as indicated by arrow 132. Subsequently, the first parts 128 indicated by large opaque squares disappear or are contracted and new small squares appear, respectively, when proceeding towards the lower portion of Figure 5 as indicated by arrow 134. Further, the second parts 130 become bolt or darker.

As is further shown in the lower portion of Figure 5, the second parts 130 are greater than the first parts 128. In order to further provide the optical flow, the first parts 128 are expanded such that large opaque squares appear, the small squares disappear and the second parts 130 are faded out when proceeding towards the middle portion of Figure 5 as indicated by arrow 136. Subsequently, the second parts 130 indicated by large opaque squares disappear or are contracted and new small squares appear when proceeding towards the upper portion of Figure 5 as indicated by arrow 138. Further, the first parts 128 are faded in or become bolt or darker. Thus, a pattern change for motions perpendicular to the display 102 is provided.

The display device 120 is further configured to vary the visual cue 114 based on the obtained self motion data. Thus, depending on the kind of detected self motion, the kind of visual cue 114 is illustrated. The display device 120 is configured to limit a shifting of the visual cue 114 to maximum 4°/s. Thus, any shifting or displacement of the illustrated visual cue 114 does not exceed a maximum resolution of a human's eye of 4°/s retinal slip. For higher velocities, a kind of saturation is thereby provided. Further, the display device 120 is configured to adjust a resolution of the visual cue 114 based on a distance of the visual cue 114 from a user's fovea. The display device 120 is configured to increase the resolution of the visual cue based on a decreasing distance of the visual cue from the user's fovea. The processing unit is configured to provide a plurality of visual cues 114 based on the obtained self motion data. The display device 120 is configured to display the plurality of visual cues 114 at the display at least in the periphery of the visual field of the user. The visual cues may be identical to one another or may be different from one another. Particularly, the display device 120 is configured to display the plurality of visual cues 114 at the display 102 in a subsequent order or at the same time.

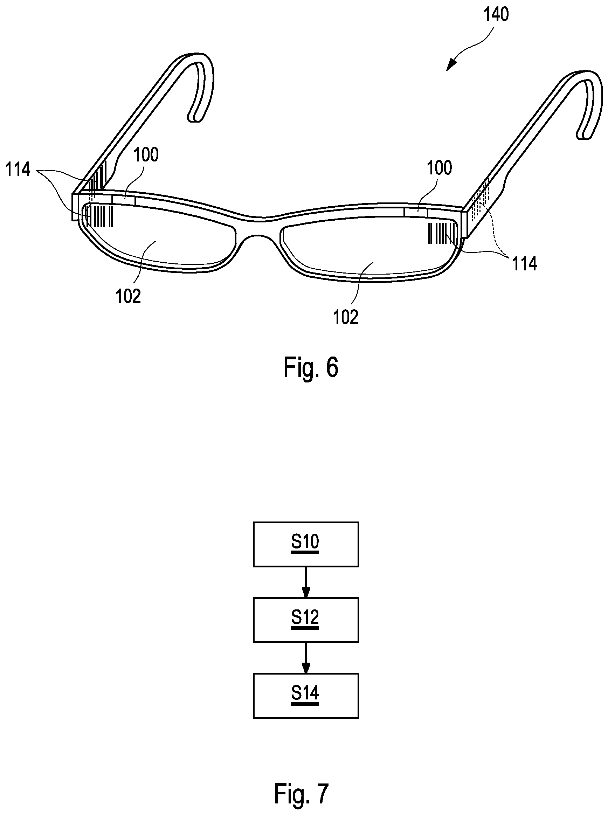

Figure 6 shows a perspective view of a visual aid 140. Figure 6 shows the opto-electronic device 100 integrated into or attached to the visual aid 140. The visual aid 140 is glasses. In this example, the display 102 is the spectacle lenses of the visual aid 140. The opto-electronic device 100 is integrated into or attached to an upper edge or frame of the spectacle lenses. It has to be noted that the opto-electronic device 100 may be located at the right and/or left upper edge or frame of the spectacle lenses. thus, also more than one opto-electronic device 100 may be present. With this set-up, the display device 120 projects at least one visual cue 114 at least onto the temple pieces of the glasses or an area adjacent or close thereto corresponding a periphery of the user's visual field. In addition, the display device 120 may project visual cues 114 onto the spectacle lenses of the glasses. In latter case, the display 102 is a transparent screen. Alternatively, the display 102 may be a user's retina. Alternatively, the opto-electronic device 100 may be a so-called stand-alone solution comprising the display 102. For example, the opto-electronic device 100 may be formed as smart glasses.

Translational motions of the user's head in the dimensions of the display 102 are viewed as horizontal or vertical shifts of the displayed pattern of the visual cue 114. Motions of the user's head perpendicular to the display 102 are viewed as contraction or expansion with corresponding fading in and out of the displayed pattern of the visual cue 114. Rotations are displayed as corresponding translations, where e.g. a clockwise rotation of the head will be displayed as backward motion on the left and forward motion of the pattern of the visual cue 114 on the right peripheral display 102.

Hereinafter, a method for providing visual orientation according to the present invention will be described. Figure 7 shows a flowchart of the method. The method may be carried out using the opto-electronic device 100 as described above. The method may be computer-implemented. The method comprises a step S10 which includes obtaining head orientation data including information on an orientation of a user's head. Step 10 may be carried out by the inertial measurement unit 108. Subsequently, the method further proceeds to step S12 which includes processing the head orientation data so as to obtain self motion data including information on the user's self motion and providing at least one visual cue 114 based on the obtained self motion data. Step S12 may be carried out by the processing unit 112. Subsequently, the method further proceeds to step S14 which includes displaying the at least one visual cue 114 at a display 102 at least in a periphery of a visual field of the user. The visual cue 114 is displayed as a virtually stationary foreground with a predetermined distance to the user. The visual cue 114 is displayed so as to form a contrast relative to a background in the visual field of the user as explained above with reference to Figure 4. The visual cue 114 provides parallax information to the user. The visual cue 114 is a space veridical orientation cue. The visual cue 114 and its shape, respectively, may be varied based on the obtained self motion data.

As described above with reference to Figure 5, the method may further comprise providing the visual cue 114 with an optical flow. This may be realized by varying a size at least of first parts 128 of the visual cue 114 and fading in or fading out at least of second parts 130 of the visual cue 114 so as to provide the optical flow. Particularly, this may be realized by gradually varying a size at least of first parts 128 of the visual cue 114 and gradually fading in or fading out at least of second parts 130 of the visual cue 114 so as to provide a gradual optical flow. As explained above, the first parts 128 and the second parts 130 of the visual cue 114 may be different from one another or may be identical to one another.

The method may further comprise limiting a shifting of the visual cue to maximum 4°/s. The method may further comprise adjusting a resolution of the visual cue 114 based on a distance of the visual cue 114 from a user's fovea. Particularly, the resolution of the visual cue 114 may be increased based on a decreasing distance of the visual cue 114 from the user's fovea. The method may further comprise providing a plurality of visual cues 114 based on the obtained self motion data, and displaying the plurality of visual cues 114 at the display 102 at least in the periphery of the visual field of the user. The visual cues 114 may be identical to one another or may be different from one another. The plurality of visual cues 114 may be displayed at the display 102 in a subsequent order or at the same time.

### List of reference numbers

- 100: opto-electronic device
- 102: display
- 104: transparent screen
- 106: sensor
- 108: inertial measurement unit
- 110: printed circuit board
- 112: processing unit
- 114: visual cue
- 116: processing device
- 118: ASIC
- 120: display device
- 122: background
- 124: bright spot
- 126: bright spot
- 128: first part
- 130: second part
- 132: arrow
- 134: arrow
- 136: arrow
- 138: arrow
- 140: visual aid
- S10: obtaining head orientation data
- S12: processing the head orientation data
- S14: displaying the at least one visual cue

## Claims

1. An opto-electronic device (100) configured to be integrated into or attached to a visual aid or formed as smart glasses, the opto-electronic device comprising
at least one sensor (106) configured to obtain head orientation data and head movement data including information on an orientation and movement of a user's head,
a processing unit (112) configured to process the head orientation data and head movement data so as to obtain self motion data including information on the user's self motion and to provide at least one visual cue (114) based on the obtained self motion data, and
a display device (120) configured to display the at least one visual cue (114) at a display (102) at least in a periphery of a visual field of the user, wherein the display (102) is a transparent screen or a user's retina, and
wherein the display device (120) is configured to display the visual cue (114) at the display (102) as a virtually stationary foreground with a predetermined distance to the user,
**characterised in that**
the display device (120) is further configured to provide the visual cue (114) with an optical flow, wherein the display device (120) is configured to vary a size at least of first parts (128) of the visual cue (114) and to fade in or fade out at least of second parts (130) of the visual cue (114) so as to provide the optical flow, wherein the display device (120) is further configured to gradually vary the size of the first parts (128) of the visual cue (114) and to fade in or fade out the second parts (130) of the visual cue (114) so as to provide a gradual optical flow, wherein motions of the user's head perpendicular to the display device (102) are viewed as contraction or expansion with corresponding fading in and out of a displayed pattern of the visual cue (114), wherein the display device (120) is configured to expand the first parts (128) of the visual cue (114) and to fade in the second parts (130) of the visual cue (114) if a distance from the visual cue (114) to the user decreases, and wherein the display device (120) is configured to contract the first parts (128) of the visual cue (114) and to fade out the second parts (130) of the visual cue (114) if a distance from the visual cue (114) to the user increases.

2. The opto-electronic device (100) according to the preceding claim, wherein the sensor (106) includes at least an inertial measurement unit (118) and/or a camera and/or a LIDAR device configured to obtain the head orientation data and head movement data.

3. The opto-electronic device (100) according to any one of the preceding claims, wherein the display device (120) is configured to display the visual cue (114) so as to form a contrast relative to a background (122) in the visual field of the user and/or wherein the visual cue (114) is configured to provide parallax information to the user, wherein the parallax information is configured to provide spatial information, and/or wherein the visual cue (114) is a space veridical orientation cue.

4. The opto-electronic device (100) according to any one of the preceding claims, wherein the visual cue (114) is at least one cue selected from the group consisting of: horizontal lines, vertical lines, a shape of a room, a shape of a tunnel, a dot pattern, a checked pattern and a pattern with depth information.

5. The opto-electronic device (100) according to any one of the preceding claims, wherein the display device (120) is configured to vary the visual cue (114) based on the obtained self motion data.

6. The opto-electronic device (100) according to any one of the preceding claims, wherein the display device (120) is configured to limit a shifting of the visual cue (114) to maximum 4°/s.

7. The opto-electronic device (100) according to any one of the preceding claims, wherein the display device (120) is configured to adjust a resolution of the visual cue (114) based on a distance of the visual cue (114) from a user's fovea, wherein the display device (120) is preferably configured to increase the resolution of the visual cue (114) based on a decreasing distance of the visual cue (114) from the user's fovea.

8. The opto-electronic device (100) according to any one of the preceding claims, wherein the processing unit (112) is configured to provide a plurality of visual cues (114) based on the obtained self motion data, and the display device (120) is configured to display the plurality of visual cues (114) at the display (102) at least in the periphery of the visual field of the user, wherein the visual cues (114) are identical to one another or are different from one another, wherein the display device (120) is preferably configured to display the plurality of visual cues (114) at the display (102) in a subsequent order or at the same time.

9. The opto-electronic device (100) according to any one of the preceding claims, wherein the display (102) is a transparent screen or a user's retina.

10. A method for providing visual orientation to a user of an opto-electronic device (100) according to any of the preceding claims, the method comprising
- obtaining head orientation data and head movement data including information on an orientation and movement of a user's head,
- processing the head orientation data and head movement data so as to obtain self motion data including information on the user's self motion and providing at least one visual cue (114) based on the obtained self motion data, and
- displaying the at least one visual cue (114) at a display (102) at least in a periphery of a visual field of the user, wherein the display (102) is a transparent screen or a user's retina, and
wherein the display device (120) is configured to display the visual cue (114) at the display (102) as a virtually stationary foreground with a predetermined distance to the user,
**characterised in that**
the display device (120) is further configured to provide the visual cue (114) with an optical flow, wherein the display device (120) is configured to vary a size at least of first parts (128) of the visual cue (114) and to fade in or fade out at least of second parts (130) of the visual cue (114) so as to provide the optical flow, wherein the display device (120) is preferably further configured to gradually vary the size of the first parts (128) of the visual cue (114) and to fade in or fade out the second parts (130) of the visual cue (114) so as to provide a gradual optical flow, wherein motions of the user's head perpendicular to the display device (102) are viewed as contraction or expansion with corresponding fading in and out of a displayed pattern of the visual cue (114), wherein the display device (120) is configured to expand the first parts (128) of the visual cue (114) and to fade in the second parts (130) of the visual cue (114) if a distance from the visual cue (114) to the user decreases, and wherein the display device (120) is configured to contract the first parts (128) of the visual cue (114) and to fade out the second parts (130) of the visual cue (114) if a distance from the visual cue (114) to the user increases.

## Patentansprüche

1. Optoelektronische Vorrichtung (100), die konfiguriert ist, um in eine visuelle Hilfe integriert oder an dieser befestigt zu werden, oder die als intelligente Brille ausgebildet ist, wobei die optoelektronische Vorrichtung umfasst:
mindestens einen Sensor (106), der konfiguriert ist zum Erhalten von Kopfausrichtungsdaten und von Kopfbewegungsdaten, die Informationen über eine Ausrichtung und eine Bewegung des Kopfes eines Benutzers beinhalten,
eine Verarbeitungseinheit (112), die konfiguriert ist zum Verarbeiten der Kopfausrichtungsdaten und der Kopfbewegungsdaten, um Eigenbewegungsdaten zu erhalten, die Informationen über die Eigenbewegung des Benutzers beinhalten, und um mindestens einen visuellen Hinweis (114) basierend auf den erhaltenen Eigenbewegungsdaten bereitzustellen, und
eine Anzeigevorrichtung (120), die konfiguriert ist, um den mindestens einen visuellen Hinweis (114) auf einer Anzeige (102) mindestens in einer Peripherie eines Blickfeldes des Benutzers anzuzeigen, wobei die Anzeige (102) ein transparenter Bildschirm oder eine Netzhaut des Benutzers ist, und
wobei die Anzeigevorrichtung (120) konfiguriert ist, um den visuellen Hinweis (114) auf der Anzeige (102) als einen virtuell stationären Vordergrund mit einem vorbestimmten Abstand zu dem Benutzer anzuzeigen, **dadurch gekennzeichnet, dass** die Anzeigevorrichtung (120) ferner konfiguriert ist, um den visuellen Hinweis (114) mit einem optischen Fluss bereitzustellen, wobei die Anzeigevorrichtung (120) konfiguriert ist zum Variieren einer Größe mindestens von ersten Teilen (128) des visuellen Hinweises (114) und zum Einblenden oder Ausblenden mindestens von zweiten Teilen (130) des visuellen Hinweises (114), um den optischen Fluss bereitzustellen, wobei die Anzeigevorrichtung (120) ferner konfiguriert ist zum allmählichen Variieren der Größe der ersten Teile (128) des visuellen Hinweises (114) und zum Einblenden oder Ausblenden der zweiten Teile (130) des visuellen Hinweises (114), um einen allmählichen optischen Fluss bereitzustellen, wobei Bewegungen des Kopfes des Benutzers senkrecht zu der Anzeigevorrichtung (102) als Verkleinerung oder Vergrößerung mit entsprechendem Ein- und Ausblenden eines angezeigten Musters des visuellen Hinweises (114) betrachtet werden, wobei die Anzeigevorrichtung (120) konfiguriert ist, um die ersten Teile (128) des visuellen Hinweises (114) zu vergrößern und die zweiten Teile (130) des visuellen Hinweises (114) einzublenden, wenn ein Abstand von dem visuellen Hinweis (114) zu dem Benutzer abnimmt, und wobei die Anzeigevorrichtung (120) konfiguriert ist, um die ersten Teile (128) des visuellen Hinweises (114) zu verkleinern und die zweiten Teile (130) des visuellen Hinweises (114) auszublenden, wenn ein Abstand von dem visuellen Hinweis (114) zu dem Benutzer zunimmt.

2. Optoelektronische Vorrichtung (100) nach dem vorhergehenden Anspruch, wobei der Sensor (106) mindestens eine Trägheitsmesseinheit (118) und/oder eine Kamera und/oder eine LIDAR-Vorrichtung beinhaltet, die konfiguriert sind, um die Kopfausrichtungsdaten und die Kopfbewegungsdaten zu erhalten.

3. Optoelektronische Vorrichtung (100) nach einem der vorhergehenden Ansprüche, wobei die Anzeigevorrichtung (120) konfiguriert ist zum Anzeigen des visuellen Hinweises (114), um einen Kontrast relativ zu einem Hintergrund (122) in dem Blickfeld des Benutzers zu bilden, und/oder wobei der visuelle Hinweis (114) konfiguriert ist, um dem Benutzer Parallaxeninformationen bereitzustellen, wobei die Parallaxeninformationen konfiguriert sind, um räumliche Informationen bereitzustellen, und/oder wobei der visuelle Hinweis (114) ein Hinweis auf eine wahrheitsgemäße räumliche Ausrichtung ist.

4. Optoelektronische Vorrichtung (100) nach einem der vorhergehenden Ansprüche, wobei der visuelle Hinweis (114) mindestens ein Hinweis ist, der aus der Gruppe ausgewählt wird, die besteht aus: horizontalen Linien, vertikalen Linien, einer Gestalt eines Raumes, einer Gestalt eines Tunnels, einem Punktmuster, einem Schachbrettmuster und einem Muster mit Tiefeninformationen.

5. Optoelektronische Vorrichtung (100) nach einem der vorhergehenden Ansprüche, wobei die Anzeigevorrichtung (120) konfiguriert ist, um den visuellen Hinweis (114) basierend auf den erhaltenen Eigenbewegungsdaten zu variieren.

6. Optoelektronische Vorrichtung (100) nach einem der vorhergehenden Ansprüche, wobei die Anzeigevorrichtung (120) konfiguriert ist, um eine Verschiebung des visuellen Hinweises (114) auf maximal 4°/s zu begrenzen.

7. Optoelektronische Vorrichtung (100) nach einem der vorhergehenden Ansprüche, wobei die Anzeigevorrichtung (120) konfiguriert ist, um eine Auflösung des visuellen Hinweises (114) basierend auf einem Abstand des visuellen Hinweises (114) von der Fovea des Benutzers anzupassen, wobei die Anzeigevorrichtung (120) vorzugsweise konfiguriert ist, um die Auflösung des visuellen Hinweises (114) basierend auf einem abnehmenden Abstand des visuellen Hinweises (114) von der Fovea des Benutzers zu vergrößern.

8. Optoelektronische Vorrichtung (100) nach einem der vorhergehenden Ansprüche, wobei die Verarbeitungseinheit (112) konfiguriert ist, um eine Vielzahl von visuellen Hinweisen (114) basierend auf den erhaltenen Eigenbewegungsdaten bereitzustellen, und wobei die Anzeigevorrichtung (120) konfiguriert ist, um die Vielzahl von visuellen Hinweisen (114) auf der Anzeige (102) mindestens in der Peripherie des Blickfeldes des Benutzers anzuzeigen, wobei die visuellen Hinweise (114) untereinander identisch sind oder voneinander verschieden sind, wobei die Anzeigevorrichtung (120) vorzugsweise konfiguriert ist, um die Vielzahl von visuellen Hinweisen (114) auf der Anzeige (102) in einer aufeinanderfolgenden Reihenfolge oder gleichzeitig anzuzeigen.

9. Optoelektronische Vorrichtung (100) nach einem der vorhergehenden Ansprüche, wobei die Anzeige (102) ein transparenter Bildschirm oder eine Netzhaut des Benutzers ist.

10. Verfahren zum Bereitstellen einer visuellen Ausrichtung für einen Benutzer einer optoelektronischen Vorrichtung (100) nach einem der vorhergehenden Ansprüche, wobei das Verfahren umfasst:
- Erhalten von Kopfausrichtungsdaten und von Kopfbewegungsdaten, die Informationen über eine Ausrichtung und eine Bewegung des Kopfes des Benutzers beinhalten,
- Verarbeiten der Kopfausrichtungsdaten und der Kopfbewegungsdaten, um Eigenbewegungsdaten zu erhalten, die Informationen über die Eigenbewegung des Benutzers zu erhalten; und Bereitstellen mindestens eines visuellen Hinweises (114) basierend auf den erhaltenen Eigenbewegungsdaten, und
- Anzeigen des mindestens einen visuellen Hinweises (114) auf einer Anzeige (102) mindestens in einer Peripherie eines Blickfeldes des Benutzers, wobei die Anzeige (102) ein transparenter Bildschirm oder eine Netzhaut des Benutzers ist, und
wobei die Anzeigevorrichtung (120) konfiguriert ist, um den visuellen Hinweis (114) auf der Anzeige (102) als einen virtuell stationären Vordergrund mit einem vorbestimmten Abstand zu dem Benutzer anzuzeigen,
**dadurch gekennzeichnet, dass** die Anzeigevorrichtung (120) ferner konfiguriert ist, um den visuellen Hinweis (114) mit einem optischen Fluss bereitzustellen, wobei die Anzeigevorrichtung (120) konfiguriert ist zum Variieren einer Größe mindestens von ersten Teilen (128) des visuellen Hinweises (114) und zum Einblenden oder Ausblenden mindestens von zweiten Teilen (130) des visuellen Hinweises (114), um den optischen Fluss bereitzustellen, wobei die Anzeigevorrichtung (120) vorzugsweise ferner konfiguriert ist zum allmählichen Variieren der Größe der ersten Teile (128) des visuellen Hinweises (114) und zum Einblenden oder Ausblenden der zweiten Teile (130) des visuellen Hinweises (114), um einen allmählichen optischen Fluss bereitzustellen, wobei Bewegungen des Kopfes des Benutzers senkrecht zu der Anzeigevorrichtung (102) als Verkleinerung oder Vergrößerung mit einem entsprechenden Ein- und Ausblenden eines angezeigten Musters des visuellen Hinweises (114) betrachtet werden, wobei die Anzeigevorrichtung (120) konfiguriert ist, um die ersten Teile (128) des visuellen Hinweises (114) zu vergrößern und die zweiten Teile (130) des visuellen Hinweises (114) einzublenden, wenn ein Abstand von dem visuellen Hinweis (114) zu dem Benutzer abnimmt, und wobei die Anzeigevorrichtung (120) konfiguriert ist, um die ersten Teile (128) des visuellen Hinweises (114) zu verkleinern und die zweiten Teile (130) des visuellen Hinweises (114) auszublenden, wenn ein Abstand von dem visuellen Hinweis (114) zu dem Benutzer zunimmt.

## Revendications

1. Dispositif opto-électronique (100) configuré pour être intégré dans ou attaché à une aide visuelle ou réalisé comme des lunettes intelligentes, le dispositif opto-électronique comprenant
au moins un capteur (106) configuré pour obtenir des données d'orientation de tête et des données de mouvement de tête comprenant des informations sur une orientation et un mouvement de la tête d'un utilisateur,
une unité de traitement (112) configurée pour traiter les données d'orientation de tête et les données de mouvement de tête de manière à obtenir des données de mouvement propre comprenant des informations sur le mouvement propre de l'utilisateur et à fournir au moins un repère visuel (114) sur la base des données de mouvement propre obtenues, et
un dispositif d'affichage (120) configuré pour afficher l'au moins un repère visuel (114) sur un afficheur (102) au moins dans une périphérie d'un champ visuel de l'utilisateur, l'afficheur (102) étant un écran transparent ou la rétine d'un utilisateur, et
le dispositif d'affichage (120) étant configuré pour afficher le repère visuel (114) sur l'afficheur (102) en un premier plan virtuellement stationnaire avec une distance prédéterminée par rapport à l'utilisateur, **caractérisé en ce que** le dispositif d'affichage (120) est en outre configuré pour fournir au repère visuel (114) un flux optique, le dispositif d'affichage (120) étant configuré pour faire varier une taille d'au moins des premières parties (128) du repère visuel (114) et pour faire apparaître en fondu ou faire disparaître en fondu l'au moins des secondes parties (130) du repère visuel (114) de façon à fournir le flux optique, le dispositif d'affichage (120) étant en outre configuré pour faire varier progressivement la taille des premières parties (128) du repère visuel (114) et pour faire apparaître en fondu ou faire disparaître en fondu les secondes parties (130) du repère visuel (114) de manière à produire un flux optique progressif, des mouvements de la tête de l'utilisateur perpendiculaires au dispositif d'affichage (102) étant vus comme une contraction ou une expansion avec une apparition et une disparition en fondu correspondantes d'un motif affiché du repère visuel (114), le dispositif d'affichage (120) étant configuré pour dilater les premières parties (128) du repère visuel (114) et pour faire apparaître en fondu les secondes parties (130) du repère visuel (114) si une distance entre le repère visuel (114) et l'utilisateur diminue, et le dispositif d'affichage (120) étant configuré pour contracter les premières parties (128) du repère visuel (114) et pour faire disparaître en fondu les secondes parties (130) du repère visuel (114) si une distance entre le repère visuel (114) et l'utilisateur augmente.

2. Dispositif opto-électronique (100) selon la revendication précédente, le capteur (106) incluant au moins une unité de mesure inertielle (118) et/ou une caméra et/ou un dispositif LIDAR configuré pour obtenir les données d'orientation de tête et les données de mouvement de tête.

3. Dispositif opto-électronique (100) selon l'une quelconque des revendications précédentes, le dispositif d'affichage (120) étant configuré pour afficher le repère visuel (114) de manière à former un contraste par rapport à un fond (122) dans le champ visuel de l'utilisateur et/ou le repère visuel (114) étant configuré pour fournir des informations de parallaxe à l'utilisateur, les informations de parallaxe étant configurées pour fournir des informations spatiales, et/ou le repère visuel (114) étant un repère d'orientation spatiale véridique.

4. Dispositif opto-électronique (100) selon l'une quelconque des revendications précédentes, le repère visuel (114) étant au moins un repère sélectionné dans le groupe constitué par : des lignes horizontales, des lignes verticales, une forme de pièce, une forme de tunnel, un motif de points, un motif en damier et un motif avec des informations de profondeur.

5. Dispositif opto-électronique (100) selon l'une quelconque des revendications précédentes, le dispositif d'affichage (120) étant configuré pour faire varier le repère visuel (114) sur la base des données de mouvement propre obtenues.

6. Dispositif opto-électronique (100) selon l'une quelconque des revendications précédentes, le dispositif d'affichage (120) étant configuré pour limiter un décalage du repère visuel (114) à un maximum de 4°/s.

7. Dispositif opto-électronique (100) selon l'une quelconque des revendications précédentes, le dispositif d'affichage (120) étant configuré pour ajuster une résolution du repère visuel (114) sur la base d'une distance du repère visuel (114) par rapport à la fovéa d'un utilisateur, le dispositif d'affichage (120) étant de préférence configuré pour augmenter la résolution du repère visuel (114) sur la base d'une distance décroissante du repère visuel (114) par rapport à la fovéa de l'utilisateur.

8. Dispositif opto-électronique (100) selon l'une quelconque des revendications précédentes, l'unité de traitement (112) étant configurée pour fournir une pluralité de repères visuels (114) sur la base des données de mouvement propre obtenues, et le dispositif d'affichage (120) étant configuré pour afficher la pluralité de repères visuels (114) sur l'afficheur (102) au moins dans la périphérie du champ visuel de l'utilisateur, les repères visuels (114) étant identiques les uns aux autres ou différents les uns des autres, le dispositif d'affichage (120) étant de préférence configuré pour afficher la pluralité de repères visuels (114) sur l'afficheur (102) dans un ordre séquentiel ou simultanément.

9. Dispositif opto-électronique (100) selon l'une quelconque des revendications précédentes, l'afficheur (102) étant un écran transparent ou la rétine d'un utilisateur.

10. Procédé pour fournir une orientation visuelle à un utilisateur d'un dispositif opto-électronique (100) selon l'une quelconque des revendications précédentes, le procédé comprenant
- l'obtention de données d'orientation de tête et des données de mouvement de tête comprenant des informations sur une orientation et un mouvement de la tête d'un utilisateur,
- le traitement des données d'orientation de tête et les données de mouvement de tête de façon à obtenir des données de mouvement propre comprenant des informations sur le mouvement propre de l'utilisateur et fournir au moins un repère visuel (114) sur la base des données de mouvement propre obtenues, et
- l'affichage de l'au moins un repère visuel (114) sur un afficheur (102) au moins dans une périphérie d'un champ visuel de l'utilisateur, l'afficheur (102) étant un écran transparent ou la rétine d'un utilisateur, et le dispositif d'affichage (120) étant configuré pour afficher le repère visuel (114) sur l'afficheur (102) en un premier plan virtuellement stationnaire avec une distance prédéterminée par rapport à l'utilisateur,
**caractérisé en ce que** le dispositif d'affichage (120) est en outre configuré pour fournir au repère visuel (114) un flux optique, le dispositif d'affichage (120) étant configuré pour faire varier une taille d'au moins des premières parties (128) du repère visuel (114) et pour faire apparaître en fondu ou faire disparaître en fondu l'au moins des secondes parties (130) du repère visuel (114) de façon à fournir le flux optique, le dispositif d'affichage (120) étant de préférence en outre configuré pour faire varier progressivement la taille des premières parties (128) du repère visuel (114) et pour faire apparaître en fondu ou faire disparaître en fondu les secondes parties (130) du repère visuel (114) de façon à fournir un flux optique progressif, des mouvements de la tête de l'utilisateur perpendiculaires au dispositif d'affichage (102) étant vus comme une contraction ou une expansion avec une apparition et une disparition en fondu correspondantes d'un motif affiché du repère visuel (114), le dispositif d'affichage (120) étant configuré pour dilater les premières parties (128) du repère visuel (114) et pour faire apparaître en fondu les secondes parties (130) du repère visuel (114) si une distance entre le repère visuel (114) et l'utilisateur diminue, et le dispositif d'affichage (120) étant configuré pour contracter les premières parties (128) du repère visuel (114) et pour faire disparaître en fondu les secondes parties (130) du repère visuel (114) si une distance entre le repère visuel (114) et l'utilisateur augmente.
